# EUROPEAN PATENT SPECIFICATION

(11) **EP 4 330 676 B1**
(45) Date of publication and mention of the grant of the patent: **11.11.2026**
(21) Application number: 22726004.9
(22) Date of filing: 26.04.2022
(51) Int. Cl.: G01N 33/575

(54) **METHODS FOR PREDICTING THE RISK OF LYMPH NODE METASTASIS AND/OR RECURRENCE OF PATIENTS SUFFERING FROM A T1 CANCER TREATED BY ENDOSCOPIC RESECTION**
VERFAHREN ZUR VORHERSAGE DES RISIKOS VON LYMPHKNOTENMETASTASEN UND/ODER REZIDIVEN BEI PATIENTEN, DIE AN EINEM DURCH ENDOSKOPISCHE RESEKTION BEHANDELTEN T1-KREBS LEIDEN
PROCÉDÉS POUR PRÉDIRE LE RISQUE DE MÉTASTASE DES GANGLIONS LYMPHATIQUES ET/OU DE RÉCIDIVE DES PATIENTS SOUFFRANT D'UN CANCER T1 TRAITÉ PAR RÉSECTION ENDOSCOPIQUE

(30) Priority: 27.04.2021 EP 21305541
(43) Date of publication of application: 06.03.2024
(73) Proprietor: Institut National de la Santé et de la Recherche Médicale (INSERM), 75013 Paris (FR); Sorbonne Université, 75006 Paris (FR); Université Paris Cité, 75006 Paris (FR); Assistance Publique-Hôpitaux de Paris (APHP), 75012 Paris (FR)
(72) Inventor: PAGES, Franck, 75908 Paris cedex 15 (FR); GALON, Jérôme, 75006 Paris (FR); CHAUSSADE, Stanislas, 75014 Paris (FR); KIRILOVSKY, Amos, 75015 Paris (FR)
(74) Representative: Inserm Transfert
(86) International application number: PCT/EP2022/060970
(87) International publication number: WO 2022/229136

(56) References cited:
- WO-A1-2017/194556
- HAASNOOT KRIJN J C ET AL: "Associations of non-pedunculated T1 colorectal adenocarcinoma outcome with consensus molecular subtypes, immunoscore, and microsatellite status: a multicenter case-cohort study", MODERN PATHOLOGY, vol. 33, no. 12, 24 June 2020 (2020-06-24) - 24 June 2020 (2020-06-24), pages 2626 - 2636, XP037304344, ISSN: 0893-3952, DOI: 10.1038/S41379-020-0598-9
- KANG JEONGHYUN ET AL: "LASSO-Based Machine Learning Algorithm for Prediction of Lymph Node Metastasis in T1 Colorectal Cancer", vol. 53, no. 3, 29 December 2020 (2020-12-29), KR, pages 773 - 783, XP055842116, ISSN: 1598-2998, Retrieved from the Internet <URL:https://www.e-crt.org/upload/pdf/crt-2020-974.pdf> DOI: 10.4143/crt.2020.974
- FRANCK PAGÈS ET AL: "International validation of the consensus Immunoscore for the classification of colon cancer: a prognostic and accuracy study", THE LANCET, vol. 391, no. 10135, 1 May 2018 (2018-05-01), AMSTERDAM, NL, pages 2128 - 2139, XP055574613, ISSN: 0140-6736, DOI: 10.1016/S0140-6736(18)30789-X
- MARLIOT FLORENCE ET AL: "Conclusion", JOURNAL FOR IMMUNOTHERAPY OF CANCER, vol. 8, no. 1, 1 May 2020 (2020-05-01), pages e000272, XP055842107, DOI: 10.1136/jitc-2019-000272
- ANGELL HELEN K. ET AL: "The Immunoscore: Colon Cancer and Beyond", CLINICAL CANCER RESEARCH, vol. 26, no. 2, 14 August 2019 (2019-08-14), US, pages 332 - 339, XP055842101, ISSN: 1078-0432, DOI: 10.1158/1078-0432.CCR-18-1851
- LEA DORDI ET AL: "A template to quantify the location and density of CD3 + and CD8 + tumor-infiltrating lymphocytes in colon cancer by digital pathology on whole slides for an objective, standardized immune score assessment", CANCER IMMUNOLOGY, IMMUNOTHERAPY, SPRINGER, BERLIN/HEIDELBERG, vol. 70, no. 7, 13 January 2021 (2021-01-13), pages 2049 - 2057, XP037479842, ISSN: 0340-7004, [retrieved on 20210113], DOI: 10.1007/S00262-020-02834-Y

## Description

### FIELD:

The present invention is in the field of medicine, in particular oncology and immunology.

### BACKGROUND:

Colorectal cancer (CRC) is the third most common cancer worldwide and the second leading cause of cancer-related death. Wide-spread application of screening colonoscopy is increasing the detection of T1 CRC from 10% in diagnostic colonoscopies to 20-40% in screening colonoscopies (Amri et al. 2013; Kubisch et al. 2016). These superficial cancers are treated with endoscopic submucosal resection as first-line treatment; offering potentially curative, organ-preserving minimally invasive treatment for early cancer. Pathological examination of the tumor determines histological criteria associated with an increased risk of lymph node metastasis; i.e. positive vertical margin, poor differentiation, vascular or lymphatic tumor emboli, tumor budding, and invasion of the submucosa > 1000 µm (Beaton et al. 2013). Therefore, their presence justifies an additional radical surgery but results in perioperative complications (38.3-45.6%), post-operative mortality 1.2%-3.6% and significant digestive, urological, and sexual functional sequelae. These pejorative histological criteria are detected in half of the patients however overall incidence of lymph node metastasis is only of 20%. This rate underline that the surgical procedure following endoscopic resection is performed by excess in 70 to 80% of the cases given that the tumor having been completely resected by endoscopy and no sign of tumor invasion is observed in the resected lymph nodes. Therefore, organ preservation could be offered to a larger number of patients if new biomarkers could complete the tumor histological evaluation and thus provide better prediction of metastatic extension to lymph nodes and relapse.

Tumor spreading is influenced by tumor characteristics and immune component. We determined an immune-based assay, "Immunoscore" (IS), in CRC. IS correlates with tumor extension and its prognostic performance has recently been consolidated in an international validation study, which we coordinated (Pagès et al. 2018). The IS has been recently included in the 2020 European Society of Medical Oncology (ESMO) Clinical Practice Guidelines for Diagnosis, Treatment, and Follow-up for Localized Colon Cancer (Argilés et al. 2020).

Very few publications on biomarkers in T1¬ CRC are available (Kandimalla et al. 2019; Ozawa et al. 2018). These results highlight the relevance of exploring the immune response in T1 tumors removed by endoscopic resection to better delineate patients eligible to organ preservation.

Recently, Kang, Jeonghyun, et al. conducted a study employing a LASSO-based machine learning algorithm to predict lymph node metastasis in T1 colorectal cancer (Kang, Jeonghyun, et al. "LASSO-based machine learning algorithm for prediction of lymph node metastasis in T1 colorectal cancer." Cancer Research and Treatment: Official Journal of Korean Cancer Association 53.3 (2021): 773-783*).* Their model integrates quantitative measurements of CD3+, CD8+, and FOXP3+ immune cell densities, alongside key histological tumor parameters, to assess the probability of lymph node involvement.

### SUMMARY OF THE INVENTION:

The present invention is defined by the claims. In particular, the present invention relates to a method of predicting the risk of lymph node metastasis and/or recurrence of a patient suffering from a T1 colorectal cancer treated by endoscopic resection and having at least one pejorative histological criterion selected from the group consisting of positive vertical margin, poor differentiation, vascular or lymphatic tumor emboli, tumor budding, and the depth of submucosal invasion > 1000 µm, comprising:
- a) quantifying in a tumor tissue sample obtained from the patient the density of CD3+ cells in center of the tumor (CT), the density of CD8+ cells in the center of the tumor (CT), the density of CD3+ cells in the invasive margin (IM), and the density of CD8+ cells in the invasive margin (IM);
- b) comparing each values obtained at step a) with a distribution of values obtained for each of said densities from a reference group of patients suffering from said cancer;
- c) determining for each values obtained at step a) for said densities the percentile of the distribution to which the values obtained at step a) correspond;
- d) calculating the arithmetic mean value or the median value of percentile
- e) comparing the arithmetic mean value or the median value of percentile obtained at step d) with a predetermined reference arithmetic mean value or a predetermined median value of percentile, and
- f) assigning a "low" "intermediate" or "high" score depending on whether the arithmetic mean value:
   - is lower (or equal) than the lowest predetermined reference arithmetic mean value of percentile ("low")
   - is comprised between the 2 predetermined reference arithmetic mean values of percentile ("intermediate")
   - is higher than the highest predetermined reference arithmetic mean value of percentile ("high"),
- g) concluding that :
   - when the patient is classified as "low", the risk of lymph node metastasis and/or recurrence is high.
   - when the patient is classified as "intermediate", the risk of lymph node metastasis and/or recurrence is intermediate
   - when the patient is classified a "high", the risk of lymph node metastasis and/or recurrence is low.

The following detailed description, figures and example do not fall under the scope of the present invention and are present for understanding and illustration purposes only.

### DETAILED DESCRIPTION:

### Main definitions:

As used herein, the term **"cancer"** refers to any cancer that can be treated by endoscopic resection. In particular the term "cancer" encompasses gastrointestinal cancers, head and neck carcinomas, and bladder cancer.

As used herein, the term **"gastrointestinal cancer"** or **"GI cancer"** is a cancer of any of the gastrointestinal tract organs or organs of the alimentary canal, i.e., mouth, esophagus, stomach, duodenum, small intestine, large intestine or colon, rectum, and anus. In particular, the gastrointestinal cancer is a colorectal cancer.

As used herein, the term **"colorectal cancer"** includes the well-accepted medical definition that defines colorectal cancer as a medical condition characterized by cancer of cells of the intestinal tract below the small intestine (i.e., the large intestine (colon), including the cecum, ascending colon, transverse colon, descending colon, sigmoid colon, and rectum). Additionally, as used herein, the term **"colorectal cancer"** also further includes medical conditions, which are characterized by cancer of cells of the duodenum and small intestine (jejunum and ileum). As used herein, the term "a microsatellite unstable colorectal cancer" refers to a colorectal cancer characterized by microsatellite instability.

As used herein, the term **"microsatellite instability"** or **"MSI"** has its general meaning and is defined as the accumulation of insertion-deletion mutations at short repetitive DNA sequences (or 'microsatellites') is a characteristic feature of cancer cells with DNA mismatch repair (MMR) deficiency. Inactivation of any of several MMR genes, including MLH1, MSH2, MSH6 and PMS2, can result in MSI. Originally, MSI was shown to correlate with germline defects in MMR genes in patients with Lynch syndrome (LS), where >90% of colorectal cancer (CRC) patients exhibit MSI. It was later recognized that MSI also occurs in ^{~}12% of sporadic CRCs occurring in patients that lack germline MMR mutations, and MSI in these patients is due to promoter methylation-induced silencing of the MLH1 gene expression. Determination of MSI status in CRC involves routine methods well known in the art.

As used herein, the term **"TNM classification"** has its general meaning in the art and refers to the classification published by the Union for International Cancer Control (UICC). The UICC TNM classification is the internationally accepted standard for cancer staging. The UICC TNM Classification is an anatomically based system that records the primary and regional nodal extent of the tumor and the absence or presence of metastases. Each individual aspect of TNM is termed as a category. T category describes the extent of the primary tumour Ta, T0, Tis, T1, T2, T3, T4, Tx N-category. N category describes the absence or presence and extent of regional lymph node metastasis N0, N1, N2, N3, Nx M-category. M category describes the absence or presence of distant metastasis M0, M1, Mx. Cancer in situ is categorized stage 0; often tumors localized to the organ of origin are staged as I or II depending on the extent, locally extensive spread, to regional nodes are staged as III, and those with distant metastasis staged as stage IV.

As used herein, the term **"T1 cancer"** thus refers to a cancer that has been classified as T1 by the TNM classification.

As used herein, the term **"endoscopic resection"** has its general meaning in the art and refers to a surgical procedure that is carried out to remove precancerous, early-stage cancer or other abnormal tissues (lesions) from the digestive tract. In particular, endoscopic mucosal resection refers to techniques such as, endoscopic mucosectomy or endoscopic submucosal dissection that are performed with a long, narrow tube equipped with a light, video camera and other instruments. The term thus encompasses endoscopic submucosal resection.

As used herein, the term **"pejorative histopathological criteria"** refers to the histological criteria that are associated with an increased risk of lymph node metastasis and thus typically comprises positive vertical margin, poor differentiation, vascular or lymphatic tumor emboli, tumor budding, and the depth of submucosal invasion > 1000 µm. The pejorative histological criteria are well known in the art and are typically described in Beaton, C., C. P. Twine, G. L. Williams, et A. G. Radcliffe. 2013. « Systematic Review and Meta-Analysis of Histopathological Factors Influencing the Risk of Lymph Node Metastasis in Early Colorectal Cancer ». Colorectal Disease 15 (7): 788-97*.*

As used herein, the term **"lymph node metastasis"** has its general meaning in the art and refers to a tumoral stage wherein the cancer has spread to at least one lymph node. The term **"lymph node"** refers to an oval- or kidney-shaped organ of the lymphatic system, present widely throughout the body including the armpit and stomach and linked by lymphatic vessels. Lymph nodes contain a diverse number of immune cells, including but not limited to B cells and T cells. Lymph nodes are important for the proper functioning of the immune system and may act as filters for foreign particles and cancer cells.

As used herein, the term **"recurrence"** refers to a return of the cancer, either locally (e.g., where it used to be before therapy) or distally (e.g., metastasis).

As used herein, the term **"risk"** in the context of the present disclosure, relates to the probability that an event will occur over a specific time period and can mean a subject's "absolute" risk or "relative" risk. Absolute risk can be measured with reference to either actual observation post-measurement for the relevant time cohort, or with reference to index values developed from statistically valid historical cohorts that have been followed for the relevant time period. Relative risk refers to the ratio of absolute risks of a subject compared either to the absolute risks of low risk cohorts or an average population risk, which can vary by how clinical risk factors are assessed. Odds ratios, the proportion of positive events to negative events for a given test result, are also commonly used (odds are according to the formula p/(l-p) where p is the probability of event and (1- p) is the probability of no event) to no- conversion. "Risk evaluation," or "evaluation of risk" in the context of the present disclosure encompasses making a prediction of the probability, odds, or likelihood that an event or disease state may occur, the rate of occurrence of the event or conversion from one disease state to another. Risk evaluation can also comprise prediction of future clinical parameters, traditional laboratory risk factor values, or other indices of relapse, either in absolute or relative terms in reference to a previously measured population. The methods of the present disclosure may be used to make continuous or categorical measurements of the risk of conversion, thus diagnosing and defining the risk spectrum of a category of subjects defined as being at risk of conversion.

As used herein, the term **"time to recurrence"** or **"TTR"** has its general meaning in the art and refers to the time to disease recurrence, where deaths without recurrence were censored at the time of death. For instance, for colorectal cancer, recurrence is typically defined only by a reappearance of primary colon cancer; second primary colon cancers or other non-colon cancers were not classified as recurrences.

As used herein, the term **"disease-free survival"** or **"DFS"** has its general meaning in the art and is defined as the time from endoscopic resection to recurrence of tumor or death, and it is typically used in the adjuvant treatment setting. The term is also known as **"relapse-free survival".**

As used herein, the expression **"short survival time"** indicates that the subject will have a survival time that will be lower than the median (or mean) observed in the general population of subjects. When the subject will have a short survival time, it is meant that the subject will have a **"poor prognosis".** Inversely, the expression **"long survival time"** indicates that the subject will have a survival time that will be higher than the median (or mean) observed in the general population of subjects. When the subject will have a long survival time, it is meant that the subject will have a **"good prognosis".**

As used herein, the term **"surgery"** applies to surgical methods undertaken for removal of cancerous tissue, including colectomy, lymph node removal, sentinel lymph node dissection. In particular, the term **"radical surgery"** also called **"radical dissection",** is surgery that is more extensive than **"conservative surgery"** and is intended to remove both a tumor and any metastases thereof, for treatment purposes.

As used herein, the term **"biomarker"** has its general meaning in the art and refers to any molecule that is detectable in a sample. Such molecules may include peptides/proteins or nucleic acids and derivatives thereof.

As used herein, the term **"parameter"** refers to any characteristic assessed when carrying out the method according to the disclosure. As used herein, the term **"parameter value"** refers to a value (a number for instance) associated to a parameter.

As used herein, the term **"score"** refers to a numeric value that is derived by combining one or more parameter in a mathematic algorithm or formula. Combining the parameters can be accomplished for example by multiplying each expression level with a defined and specified coefficient and summing up such products to yield a score. The score may be determined by a scoring system that can be a continuous scoring system or a non-continuous scoring system.

As used herein, the term **"scoring system"** refers to any method in which the application of an agreed numerical scale is used as a means of estimating the degree of a response (i.e. the immune response or the clinical response).

As used herein, the term **"automated scoring system"** means that the scoring system is in part or entirely controlled and carried out by machine (e.g. a computer) and, hereby limiting the human input.

As used herein, the term **"continuous scoring system"** refers to a scoring system into which one or more variables that is input are continuous. The term **"continuous"** indicates that the variable can take on any value between its minimum value and its maximum value. In particular, the value input into the continuous scoring system is the actual magnitude of the variable. In particular, the value input into the continuous scoring system is the absolute value of the variable. In particular, the value input into the continuous scoring system is a normalized value of the variable. Conversely, the term **"non-continuous scoring system"** or **"binary scoring system"** each variable is assigned to a pre-determined "bin" (for example, "high", "intermediate" or "low"). For example, if the variable being assessed is a density of CD3+ T-cells, in a continuous scoring system, the value input into the function is the density of CD3+ T-cells and in a non-continuous scoring system, the density value is first analyzed to determine whether it falls into a "high density", a "medium density" or a "low density". Thus, consider two samples, a first having a density of 1000 CD3+ cells/mm² and a second having a density of 500 CD3+ cells/mm², the values input into a continuous scoring system would be 500 and 700, respectively and the values input into a non-continuous scoring system would depend on the bin in which they fall. If the "high bin" encompasses both 500 and 1000 cells/mm2, then a value of 1 would be input into the non-continuous scoring system for each sample. If the cut-off value between "high" and "low" bins fell somewhere between 500 and 1000 cells/mm², then a value of "high" would be input into the non-continuous scoring system for the first sample, and a value of "low" would be input into the non-continuous scoring system for the second sample. A useful way of determining such cut-off value is to construct a receiver-operator curve (ROC curve) on the basis of all conceivable cut-off values, determine the single point on the ROC curve with the closest proximity to the upper left corner (0/1) in the ROC plot. Obviously, most of the time cut-off values will be determined by less formalized procedures by choosing the combination of sensitivity and specificity determined by such cut-off value providing the most beneficial medical information to the problem investigated. Please note that these values are intended to illustrate the difference between a continuous scoring system and a non-continuous scoring system, and should not be construed as in any way limiting the scope of the disclosure unless recited in a claim.

As used herein, the term **"immunoscore"** refers to the combination of CD3+ and CD8+ T cell densities determined in the tumor (i.e. centre) and its invasive margin obtained from the patient as described in the EXAMPLE. Immunoscore^{®} is a registered trademark from INSERM (Institut National de la Sante et de la Recherche Médicale) - France). In particular, Inserm is the owner of the trademark "IMMUNOSCORE" duly protected in United States of America through the International Registration no. 1146519 in classes 01, 05, 09, 10, 42 and 44.

As used herein, the term **"percentile"** has its general meaning in the art and refers to a measure used in statistics indicating the value below which a given percentage of observations in a group of observations falls. For example, the 20th percentile is the value (or score) below which 20% of the observations may be found. Equivalently, 80% of the observations are found above the 20th percentile. The term percentile and the related term percentile rank are often used in the reporting of scores from norm-referenced tests. For example, if a score is at the 86th percentile, where 86 is the percentile rank, it is equal to the value below which 86% of the observations may be found (carefully contrast with in the 86th percentile, which means the score is at or below the value below which 86% of the observations may be found-every score is in the 100th percentile). The 25th percentile is also known as the first quartile (Q1), the 50th percentile as the median or second quartile (Q2), and the 75th percentile as the third quartile (Q3). In general, percentiles and quartiles are specific types of quantiles.

As used herein, the term **"arithmetic mean value"** has its general meaning in the art and refers to the quantity obtained by summing two or more numbers or variables and then dividing by the number of numbers or variables.

As used herein, the term **"median value"** has its general meaning in the art and refers to the value that separated the higher half from the lower half of a data sample, a population or a probability distribution. For a data set, it may be thought of as "the middle" value.

As used herein, the term **"digital pathology"** is a sub-field of pathology that focuses on data management based on information generated from digitized specimen slides. It will be understood that such images will have features in the images representing tissue features, such as shape and color, and texture. These features can be extracted in quantitative form through the use of computer-based technology.

As used herein, the term **"tumor tissue sample"** means any tissue tumor sample derived from the patient. Said tissue sample is obtained for the purpose of the in vitro evaluation. Preferably, the tumor tissue sample results from the resected tumor and encompasses the center of the tumor **(or core),** and the tissue directly surrounding the tumor **("invasive margin").** The tumor tissue sample can, of course, be subjected to a variety of well-known post-collection preparative and storage techniques (e.g., fixation, storage, freezing, etc.). The sample can be fresh, frozen, fixed (e.g., formalin fixed), or embedded (e.g., paraffin embedded). Typically the tumor tissue sample is fixed in formalin and embedded in a rigid fixative, such as paraffin (wax) or epoxy, which is placed in a mould and later hardened to produce a block which is readily cut. Thin slices of material can be then prepared using a microtome, placed on a glass slide and submitted e.g. to immunohistochemistry (using an IHC automate such as BenchMark^{®} XT, for obtaining stained slides).

### Methods of the present disclosure:

The present disclosure relates to a method of predicting the risk of lymph node metastasis and/or recurrence of a patient suffering from a T1 cancer treated by endoscopic resection comprising quantifying in tumor sample obtained from the patient the density of CD3+ cells in center of the tumor (CT), the density of CD8+ cells in the center of the tumor (CT), the density of CD3+ cells in the invasive margin (IM), and the density of CD8+ cells in the invasive margin (IM). In particular, the patient suffers from a T1 gastrointestinal cancer. In particular, the patient suffers from a T1 colorectal cancer.

In particular, the method of the present disclosure is particularly suitable for predicting time to recurrence.

In particular, the method of the present disclosure is particularly suitable for predicting the disease-free survival.

In particular, the patient has at least one pejorative histological criteria.

In particular, the method of the present disclosure implements a scoring system that inputs the quantification values of the density of CD3+ cells in center of the tumor (CT), the density of CD8+ cells in the center of the tumor (CT), the density of CD3+ cells in the invasive margin (IM), and the density of CD8+ cells in the invasive margin (IM).

In particular, the immune response is assessed by a continuous scoring system that involves the steps of:
a) quantifying in the tumor tissue sample the density of CD3+ cells in center of the tumor (CT), the density of CD8+ cells in the center of the tumor (CT), the density of CD3+ cells in the invasive margin (IM), and the density of CD8+ cells in the invasive margin (IM);
b) comparing each values obtained at step a) with a distribution of values obtained for each of said densities from a reference group of patients suffering from said cancer;
c) determining for each values obtained at step a) for said densities the percentile of the distribution to which the values obtained at step a) correspond;
d) calculating the arithmetic mean value or the median value of percentiles.

Typically the higher is the arithmetic mean value or the median value of percentile, the lower is the risk of lymph node metastasis and/or recurrence for the patient and conversely, the lower is the arithmetic mean value or the median value of percentile, the higher is the risk of lymph node metastasis and/or recurrence for the patient. More particularly, the higher is the arithmetic mean value or the median value of percentile, the longer will be the disease-free survival time (good prognosis), and conversely the lower is the arithmetic mean value or the median value of percentile, the shorter will be the disease-free survival time (poor prognosis).

In particular, the scoring system is a non-continuous system. In particular, the scoring system is a non-continuous system wherein the absolute quantification values of the density of CD3+ cells in center of the tumor (CT), the density of CD8+ cells in the center of the tumor (CT), the density of CD3+ cells in the invasive margin (IM), and the density of CD8+ cells in the invasive margin (IM) is assigned to a predetermined bin. In particular, the scoring system is a non-continuous system wherein the absolute quantification values of cell densities determined in the tumor tissue sample obtained from the patient are assigned to a "high" or "low" bin. In particular, the scoring system is a non-continuous system wherein the absolute quantification values of CD3+ and CD8+ cell densities determined in the tumor sample obtained from the patient are assigned to a "high" or "low" bin. Accordingly, the cell density value is thus compared to a predetermined reference value and thus is assigned to a "low" or "high" bin depending on whether the cell density is lower or higher than the predetermined reference value. The scoring system thus outputs a non-continuous variable such as "low", "medium" and "high".

In particular, the immune response is assessed by a non-continuous scoring system that involves the steps of:
a) quantifying in the tumor tissue sample the density of CD3+ cells in center of the tumor (CT), the density of CD8+ cells in the center of the tumor (CT), the density of CD3+ cells in the invasive margin (IM), and the density of CD8+ cells in the invasive margin (IM);
b) comparing each values obtained at step a) with a distribution of values obtained for each of said densities from a reference group of patients suffering from said cancer;
c) determining for each values obtained at step a) for said densities the percentile of the distribution to which the values obtained at step a) correspond;
d) calculating the arithmetic mean value or the median value of percentile
e) comparing the arithmetic mean value or the median value of percentile obtained at step d) with a predetermined reference value, and
f) assigning a "low" or "high" score depending on whether the arithmetic mean value or the median value of percentile is respectively lower/equal or higher than the predetermined reference value.

In particular, the immune response is assessed by a non-continuous scoring system that involves the steps of:
a) quantifying in the tumor tissue sample the density of CD3+ cells in center of the tumor (CT), the density of CD8+ cells in the center of the tumor (CT), the density of CD3+ cells in the invasive margin (IM), and the density of CD8+ cells in the invasive margin (IM);
b) comparing each values obtained at step a) with a distribution of values obtained for each of said densities from a reference group of patients suffering from said cancer;
c) determining for each values obtained at step a) for said densities the percentile of the distribution to which the values obtained at step a) correspond;
d) calculating the arithmetic mean value or the median value of percentile
e) comparing the arithmetic mean value or the median value of percentile obtained at step d) with a predetermined reference arithmetic mean value or a predetermined median value of percentile, and
f) assigning a "low" "intermediate" or "high" score depending on whether the arithmetic mean value:
   - is lower (or equal) than the lowest predetermined reference arithmetic mean value of percentile ("low")
   - is comprised between the 2 predetermined reference arithmetic mean values of percentile ("intermediate")
   - is higher than the highest predetermined reference arithmetic mean value of percentile ("high").

Typically, when the patient is classified as "low", the risk of lymph node metastasis and/or recurrence is high. When the patient is classified as "intermediate", the risk of lymph node metastasis and/or recurrence is intermediate. When the patient is classified a "high", the risk of lymph node metastasis and/or recurrence is low. More particularly, the patient classified as "low" will have a short disease-free survival time, the patient classified as "intermediate" will have an intermediate disease-free survival time, and the patient classified as "high" will have a long disease-free survival time.

In particular, the non-continuous scoring system is Immunoscore as described in the EXAMPLE.

In particular, step f) comprises classifying the patient in groups (Low, Intermediate, or High Immunoscore (IS)) as follows:
- Low IS corresponds to a mean percentile below or equal to 25%,
- Intermediate IS corresponds to a mean percentile between 25% and 70%,
- High IS corresponds to a mean percentile above 70%;
wherein the mean percentile refers to the mean percentile of the 4 individual percentiles of the density of CD3+ cells in center of the tumor (CT), the density of CD8+ cells in the center of the tumor (CT), the density of CD3+ cells in the invasive margin (IM), and the density of CD8+ cells in the invasive margin (IM).

In particular, step f) comprises classifying the patient in groups (Low, Intermediate, or High Immunoscore (IS)) as follows:
- Low IS corresponds to a mean percentile below or equal to 33%,
- Intermediate IS corresponds to a mean percentile between 33% and 66%,
- High IS corresponds to a mean percentile above 66%;
wherein the mean percentile refers to the mean percentile of the 4 individual percentiles of the density of CD3+ cells in center of the tumor (CT), the density of CD8+ cells in the center of the tumor (CT), the density of CD3+ cells in the invasive margin (IM), and the density of CD8+ cells in the invasive margin (IM).

In particular, the scoring system involves digital pathology as described herein after.

In particular, the scoring system is an automated scoring system.

### Methods for quantifying the cell densities:

In particular, the density of CD3+ cells in center of the tumor (CT), the density of CD8+ cells in the center of the tumor (CT), the density of CD3+ cells in the invasive margin (IM), and the density of CD8+ cells in the invasive margin (IM) are quantified with any one of the immunohistochemistry methods known in the art.

Typically, for further analysis, one thin section of the tumor, is firstly incubated with labeled antibodies directed against one immune marker of interest (CD3 or CD8). After washing, the labeled antibodies that are bound to said immune marker of interest are revealed by the appropriate technique, depending of the kind of label is borne by the labeled antibody, e.g. radioactive, fluorescent or enzyme label. Multiple labelling can be performed simultaneously.

Immunohistochemistry typically includes the following steps i) fixing the tumor biopsy sample with formalin, ii) embedding said tumor biopsy sample in paraffin, iii) cutting said tumor biopsy sample into sections for staining, iv) incubating said sections with the binding partner specific for the immune marker (CD3 or CD8), v) rinsing said sections, vi) incubating said section with a secondary antibody typically biotinylated and vii) revealing the antigen-antibody complex typically with avidin-biotin-peroxidase complex. Accordingly, the tumor biopsy sample is firstly incubated with the binding partners having for the immune marker (CD3 or CD8). After washing, the labeled antibodies that are bound to the immune marker (CD3 or CD8) are revealed by the appropriate technique, depending of the kind of label is borne by the labeled antibody, e.g. radioactive, fluorescent or enzyme label. Multiple labelling can be performed simultaneously. Alternatively, the method of the present disclosure may use a secondary antibody coupled to an amplification system (to intensify staining signal) and enzymatic molecules. Such coupled secondary antibodies are commercially available, e.g. from Dako, EnVision system. Counterstaining may be used, e.g. Hematoxylin & Eosin, DAPI, Hoechst. Other staining methods may be accomplished using any suitable method or system as would be apparent to one of skill in the art, including automated, semi-automated or manual systems.

For example, one or more labels can be attached to the antibody, thereby permitting detection of the target protein (i.e. the immune markers). Exemplary labels include radioactive isotopes, fluorophores, ligands, chemiluminescent agents, enzymes, and combinations thereof. Nonlimiting examples of labels that can be conjugated to primary and/or secondary affinity ligands include fluorescent dyes or metals (e.g. fluorescein, rhodamine, phycoerythrin, fluorescamine), chromophoric dyes (e.g. rhodopsin), chemiluminescent compounds (e.g. luminal, imidazole) and bioluminescent proteins (e.g. luciferin, luciferase), haptens (e.g. biotin). A variety of other useful fluorescers and chromophores are described in Stryer L (1968) Science 162:526-533 and Brand L and Gohlke J R (1972) Annu. Rev. Biochem. 41:843-868. Affinity ligands can also be labeled with enzymes (e.g. horseradish peroxidase, alkaline phosphatase, beta-lactamase), radioisotopes (e.g. ³H, ¹⁴C, ³²P, ³⁵S or ¹²⁵I) and particles (e.g. gold). The different types of labels can be conjugated to an affinity ligand using various chemistries, e.g. the amine reaction or the thiol reaction. However, other reactive groups than amines and thiols can be used, e.g. aldehydes, carboxylic acids and glutamine. Various enzymatic staining methods are known in the art for detecting a protein of interest. For example, enzymatic interactions can be visualized using different enzymes such as peroxidase, alkaline phosphatase, or different chromogens such as DAB, AEC or Fast Red. In particular, the label is a quantum dot. For example, Quantum dots (Qdots) are becoming increasingly useful in a growing list of applications including immunohistochemistry, flow cytometry, and plate-based assays, and may therefore be used in conjunction with this disclosure. Qdot nanocrystals have unique optical properties including an extremely bright signal for sensitivity and quantitation; high photostability for imaging and analysis. A single excitation source is needed, and a growing range of conjugates makes them useful in a wide range of cell-based applications. Qdot Bioconjugates are characterized by quantum yields comparable to the brightest traditional dyes available. Additionally, these quantum dot-based fluorophores absorb 10-1000 times more light than traditional dyes. The emission from the underlying Qdot quantum dots is narrow and symmetric which means overlap with other colors is minimized, resulting in minimal bleed through into adjacent detection channels and attenuated crosstalk, in spite of the fact that many more colors can be used simultaneously. In other examples, the antibody can be conjugated to peptides or proteins that can be detected via a labeled binding partner or antibody. In an indirect IHC assay, a secondary antibody or second binding partner is necessary to detect the binding of the first binding partner, as it is not labeled.

In particular, the resulting stained specimens are each imaged using a system for viewing the detectable signal and acquiring an image, such as a digital image of the staining. Methods for image acquisition are well known to one of skill in the art. For example, once the sample has been stained, any optical or non-optical imaging device can be used to detect the stain or biomarker label, such as, for example, upright or inverted optical microscopes, scanning confocal microscopes, cameras, scanning or tunneling electron microscopes, canning probe microscopes and imaging infrared detectors. In some examples, the image can be captured digitally. The obtained images can then be used for quantitatively or semi-quantitatively determining the amount of the immune checkpoint protein in the sample, or the absolute number of cells positive for the maker of interest, or the surface of cells positive for the maker of interest. Various automated sample processing, scanning and analysis systems suitable for use with IHC are available in the art. Such systems can include automated staining and microscopic scanning, computerized image analysis, serial section comparison (to control for variation in the orientation and size of a sample), digital report generation, and archiving and tracking of samples (such as slides on which tissue sections are placed). Cellular imaging systems are commercially available that combine conventional light microscopes with digital image processing systems to perform quantitative analysis on cells and tissues, including immunostained samples. See, e.g., the CAS-200 system (Becton, Dickinson & Co.). In particular, detection can be made manually or by image processing techniques involving computer processors and software. Using such software, for example, the images can be configured, calibrated, standardized and/or validated based on factors including, for example, stain quality or stain intensity, using procedures known to one of skill in the art (see e.g., published U.S. Patent Publication No. US20100136549). The image can be quantitatively or semi-quantitatively analyzed and scored based on staining intensity of the sample. Quantitative or semi-quantitative histochemistry refers to method of scanning and scoring samples that have undergone histochemistry, to identify and quantify the presence of the specified biomarker (i.e. immune checkpoint protein). Quantitative or semi-quantitative methods can employ imaging software to detect staining densities or amount of staining or methods of detecting staining by the human eye, where a trained operator ranks results numerically. For example, images can be quantitatively analyzed using a pixel count algorithms and tissue recognition pattern (e.g. Aperio Spectrum Software, Automated QUantitatative Analysis platform (AQUA^{®} platform), or Tribvn with Ilastic and Calopix software, Halo software (indicalabs)), and other standard methods that measure or quantitate or semi-quantitate the degree of staining; see e.g., U.S. Pat. No. 8,023,714; U.S. Pat. No. 7,257,268; U.S. Pat. No. 7,219,016; U.S. Pat. No. 7,646,905; published U.S. Patent Publication No. US20100136549 and 20110111435; Camp et al. (2002) Nature Medicine, 8:1323-1327; Bacus et al. (1997) Analyt Quant Cytol Histol, 19:316-328). A ratio of strong positive stain (such as brown stain) to the sum of total stained area can be calculated and scored. The amount of the detected biomarker (i.e. the immune checkpoint protein) is quantified and given as a percentage of positive pixels and/or a score. For example, the amount can be quantified as a percentage of positive pixels. In some examples, the amount is quantified as the percentage of area stained, e.g., the percentage of positive pixels. For example, a sample can have at least or about at least or about 0, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 40%, 45%, 50%, 55%, 60%, 65%, 70%, 75%, 80%, 85%, 90%, 95% or more positive pixels as compared to the total staining area. For example, the amount can be quantified as an absolute number of cells positive for the maker of interest. In particular, a score is given to the sample that is a numerical representation of the intensity or amount of the histochemical staining of the sample, and represents the amount of target biomarker (e.g., the immune checkpoint protein) present in the sample. Optical density or percentage area values can be given a scaled score, for example on an integer scale.

Thus, In particular, the method of the present disclosure comprises the steps consisting in i) providing one or more immunostained slices of tissue section obtained by an automated slide-staining system by using a binding partner capable of selectively interacting with the immune marker, ii) proceeding to digitalisation of the slides of step i) by high resolution scan capture, iii) detecting the slice of tissue section on the digital picture iv) providing a size reference grid with uniformly distributed units having a same surface, said grid being adapted to the size of the tissue section to be analysed, and v) detecting, quantifying and measuring intensity or the absolute number of stained cells in each unit.

### Applications of the method of the present disclosure:

The method of the present disclosure is particularly suitable for orientating the clinical decisions after the endoscopic resection.

In T1 colorectal cancers treated by endoscopic resection, secondary major surgical resection (colectomy or rectal amputation) is usually performed when only one pejorative histological criteria is present, as the overall incidence of lymph node metastasis is of 20%. This rate underline that the surgical procedure following endoscopic resection is performed by excess in 70 to 80% of the cases (redundant) given that the tumor having been completely resected by endoscopy and no sign of tumor invasion is observed in the resected lymph nodes. This surgical procedure is associated with perioperative complications and significant digestive, urological, and sexual functional sequelae.

Organ preservation could be offered to a larger number of patients if Immunoscore is determined to complete the tumor histological evaluation and thus provide better prediction of metastatic extension to lymph nodes and relapse. In particular, in patients with Immunoscore High, it is concluded that the patient will have a low risk of lymph node metastasis and/or recurrence (i.e. will have a long disease-free survival time), an organ preservation strategy may be decided, even if the patient has at least one pejorative histological criteria.

The method of the present disclosure thus provides the advantage of identifying a certain subgroup of patients that have exceptionally good clinical outcomes while preserving quality of life. Driven by patient demand and interest in preserving quality of life, the method of the present disclosure provides a powerful tool for implementing an organ preservation strategy (i.e. avoiding surgery such as radical surgery) as well as a watch and wait strategy so that the quality of life of the patient may be preserved. More particularly, the method of the present disclosure is particularly suitable for managing the risk of perioperative morbidity and mortality, particularly for the elderly. More particularly, the method of the present disclosure is particularly suitable for preventing ample loss of anorectal, sexual, and urinary function, which eventually leads to poor quality of life in patients suffering from colorectal cancer.

The disclosure will be further illustrated by the following figures and examples. However, these examples and figures should not be interpreted in any way as limiting the scope of the present disclosure.

### FIGURES:

**Figure 1****.** Mean percentile of IS of T1 tumors with pejorative histologic criteria (n ≥ 1): lymph node metastasis and/or relapse status.
**Figure 2****.** IS status of T1 tumors with pejorative histologic criteria (n ≥ 1): lymph node status.
**Figure 3****.** IS status of T1 tumors with pejorative histologic criteria (n ≥ 1): lymph node and relapse status.
**Figure 4****.** IS status of T1 tumors with one pejorative histologic criteria: lymph node and relapse status.
**Figure 5****.** Event-free survival (lymph node metastasis or relapse) according to IS categories in patients with T1 tumors with pejorative histologic criteria (n ≥ 1).
**Figure 6****.** Event-free survival (lymph node metastasis or relapse) according to IS categories in patients with T1 tumors with only 1 pejorative histologic criteria.
**Figure 7****.** 3-years event free (lymph node invasion or relapse) survival prediction under a cox model for T1 tumors with 1 or 1 or more histologic pejorative criteria.
**Figure 8****.** Univariate analysis in patients with 1 or more pejorative histologic criteria (event = lymph node metastasis or relapse).
**Figure 9****.** Multivariate analysis in patients with 1 or more pejorative histologic criteria (event = lymph node metastasis or relapse).

### EXAMPLE:

### Methods

**Patient population:** French multi-institutional cohort of patients with stage T1 CRC treated by primary endoscopic resection and additional radical surgery (n=54). All patients presented at least one pejorative histological criteria. 12 patients with lymph node metastasis and 3 relapses were detected.

**Clinical outcomes:** Patients were compared according to the presence or absence of lymph node metastasis and/or relapse (15 events on 54 patients (27.8%).

**Immunohistochemistry:** Primary endoscopic resections were retrieved from all centers. Two formalin-fixed paraffin-embedded (FFPE) tumor tissue sections of 4 µm were processed for immunochemistry with antibodies against CD3+ (clone HDx2; HalioDx) and CD8+ (clone HDx1; HalioDx) according to the previously described protocol (Pagès et al 2020) revealed with the Ultraview Universal DAB IHC Detection Kit (Ventana, Tuscon, AZ, USA), and counterstained with Mayer's hematoxylin.

**Immunoscore (IS) determination:** methodology used to determine the Immunoscore (IS) in the international validation cohort of IS in colon cancers which have shown a strong inter-observer reproducibility (Pagès et al. 2018).

**Statistical analysis:** Survival univariate analyses were performed using the log-rank test and the Cox proportional hazards model. Survival curves were estimated by the Kaplan-Meier method. The log-rank test for trend from the survminer R package was performed to detect ordered differences in survival curves. Multivariate survival analyses were performed with Cox proportional hazards model to test the simultaneous influence of all covariates. The proportional hazards assumption (PHA) for each covariate was tested using the cox.zph function. The ordinal association between IS and lymph node metastasis and/or relapse event was assessed using a unilateral linear-by-linear association test. Mean score (IS) difference between presence or absence of lymph node metastasis and/or relapse patients was assessed with T test. P values <0.05 were considered statistically significant.

### Results

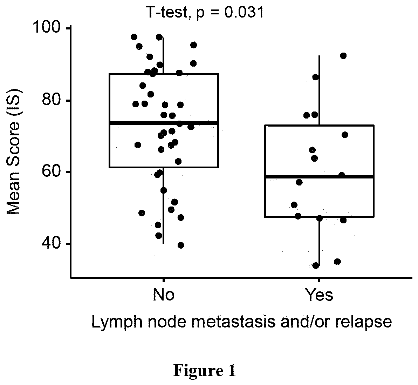

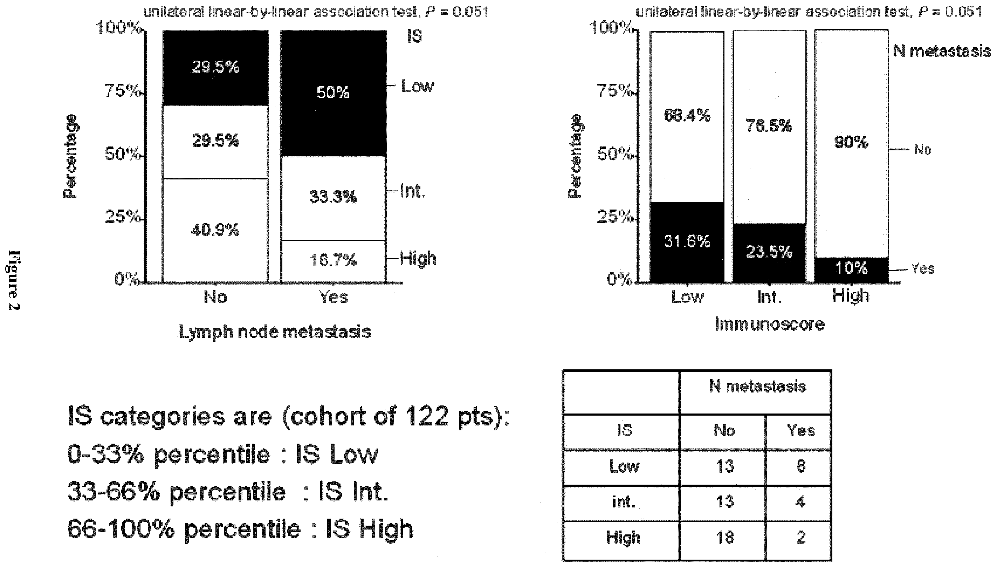

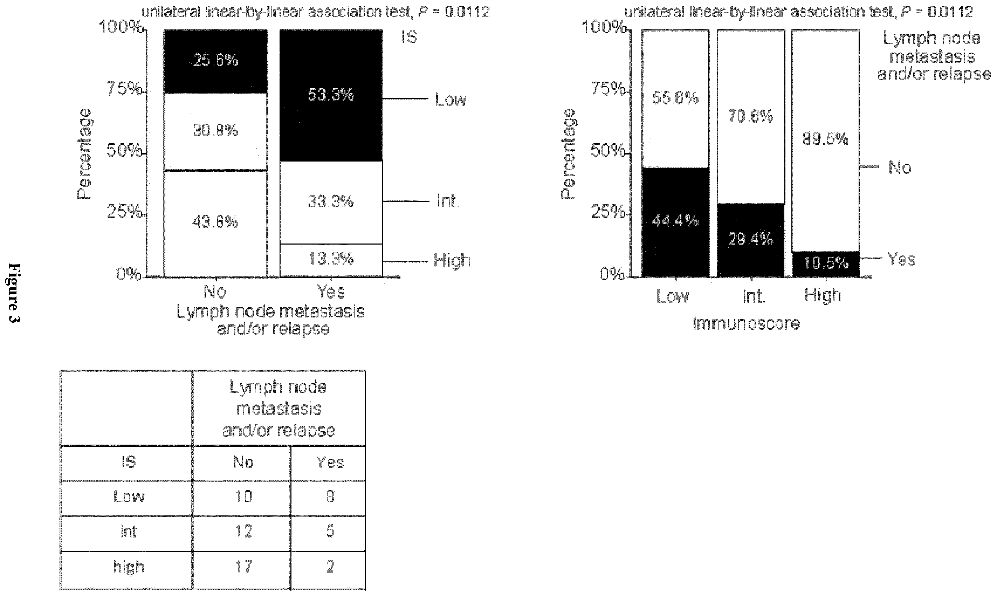

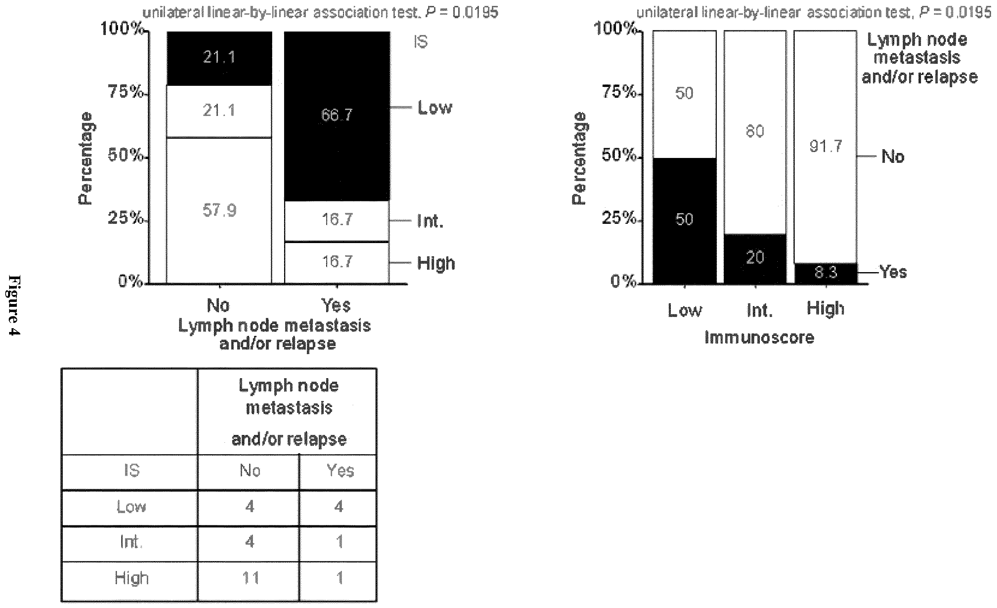

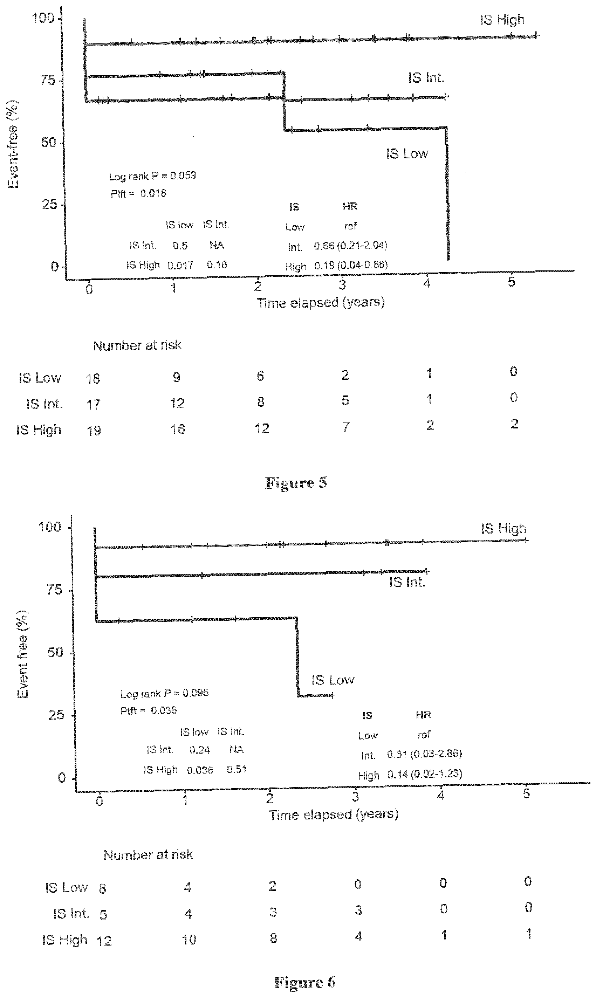

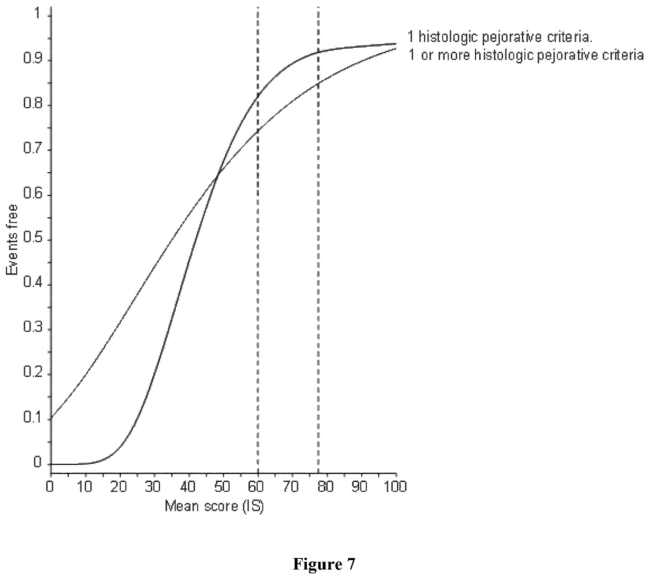

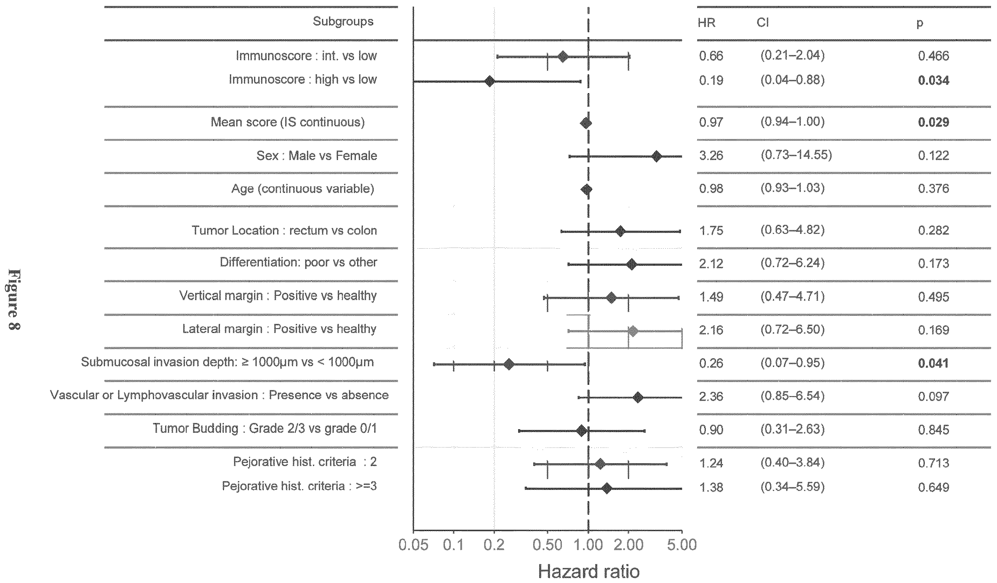

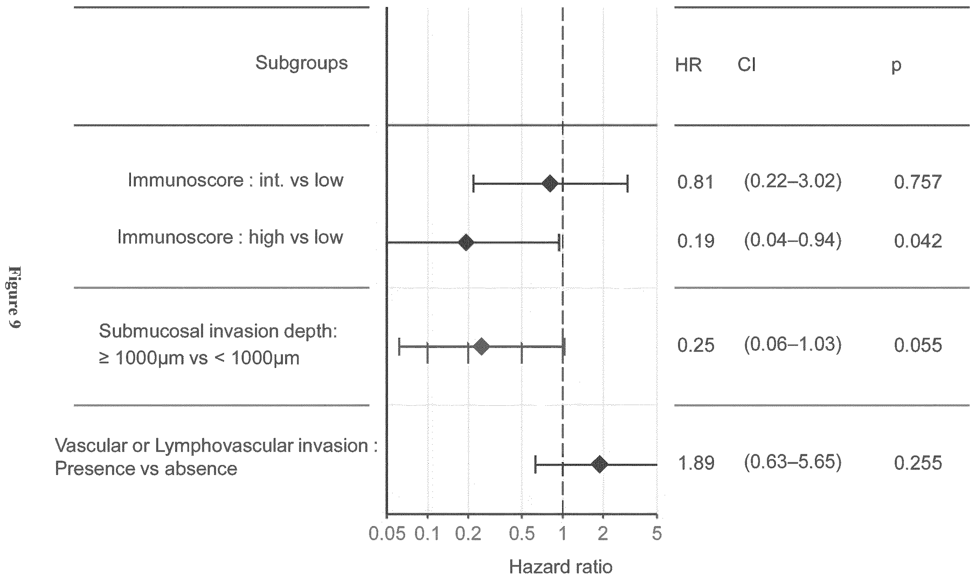

An analysis of the 54 patients with T1 CRC treated by endoscopic resection and additional radical surgery presenting at least one pejorative histological criteria showed that patients without lymph node metastases and/or recurrence compared to those with, have a significantly higher IS expressed as a continuous variable (mean score) (p=0.031; **Figure 1****).** Significant difference in IS distributions (High vs Intermediate vs Low; p=0.051) between patients with or without lymph node metastasis is observed **(****Figure 2****),** and the prognostic accuracy further increases when relapse events are taking into account (p =0.011; **Figure 3****).** Among patients with IS High only 10.5% had a lymph node metastasis, while 44.4% have lymph node metastasis and/or recurrence among patients with IS Low. Even if only one pejorative histological criteria was detected, High IS was associated with less dissemination (p =0.02; **Figure 4****).** Event-free survival (lymph node metastasis and/or recurrence; P (test for trend) = 0.018) differed between patients' groups classified by IS **(****Figure 5****).** This result is maintained when only one pejorative histological criteria was detected (P (test for trend) = 0.036; **Figure 6****).** The risk of lymph node metastasis and/or recurrence gradually decreased along with decreasing IS expressed as a mean score **(****Figure 7****).** In univariate survival analysis IS as categories score or mean percentile score and submucosal invasion depth criteria were significant **(****Figure 8****).** However, in multivariable cox model, only IS remain significant **(****Figure 9****).**

### Conclusions:

In patients with T1 tumor resected by endoscopy, eligible to radical surgery (one or more pejorative histologic criteria), IS performed on the tumor resected by endoscopy, predicts the lymph node status (metastasis or not) and/or the event of relapse. This could help to better select patients eligible to a secondary radical surgery and consequently avoid numerous unnecessary radical surgery.

### REFERENCES:

Throughout this application, various references describe the state of the art to which this disclosure pertains.

## Claims

1. A method of predicting the risk of lymph node metastasis and/or recurrence of a patient suffering from a T1 colorectal cancer treated by endoscopic resection and having at least one pejorative histological criterion selected from the group consisting of positive vertical margin, poor differentiation, vascular or lymphatic tumor emboli, tumor budding, and the depth of submucosal invasion > 1000 µm, comprising:
- a) quantifying in a tumor tissue sample obtained from the patient the density of CD3+ cells in center of the tumor (CT), the density of CD8+ cells in the center of the tumor (CT), the density of CD3+ cells in the invasive margin (IM), and the density of CD8+ cells in the invasive margin (IM);
- b) comparing each values obtained at step a) with a distribution of values obtained for each of said densities from a reference group of patients suffering from said cancer;
- c) determining for each values obtained at step a) for said densities the percentile of the distribution to which the values obtained at step a) correspond;
- d) calculating the arithmetic mean value or the median value of percentile
- e) comparing the arithmetic mean value or the median value of percentile obtained at step d) with a predetermined reference arithmetic mean value or a predetermined median value of percentile, and
- f) assigning a "low" "intermediate" or "high" score depending on whether the arithmetic mean value:
- is lower (or equal) than the lowest predetermined reference arithmetic mean value of percentile ("low")
- is comprised between the 2 predetermined reference arithmetic mean values of percentile ("intermediate")
- is higher than the highest predetermined reference arithmetic mean value of percentile ("high"),
- g) concluding that :
- when the patient is classified as "low", the risk of lymph node metastasis and/or recurrence is high.
- when the patient is classified as "intermediate", the risk of lymph node metastasis and/or recurrence is intermediate
- when the patient is classified a "high", the risk of lymph node metastasis and/or recurrence is low.

2. The method of claim 1, wherein the method is suitable for predicting time to recurrence.

3. The method of claim 1, wherein the method is suitable for predicting the disease-free survival.

## Patentansprüche

1. Verfahren zum Vorhersagen des Risikos von Lymphknotenmetastasen und/oder Rezidiv eines Patienten, der an einem T1-Kolorektalkarzinom leidet, das durch endoskopische Resektion behandelt wurde und mindestens ein ungünstiges histologisches Kriterium aufweist, ausgewählt aus der Gruppe bestehend aus positivem vertikalem Rand, mangelhafter Differenzierung, vaskulären oder lymphatischen Tumoremboli, Tumor-Budding und der Tiefe von submuköser Invasion > 1000 µm, umfassend:
- a) Quantifizieren in einer Tumorgewebeprobe, die von dem Patienten erhalten wurde, der Dichte von CD3+-Zellen im Zentrum des Tumors (CT), der Dichte von CD8+-Zellen im Zentrum des Tumors (CT), der Dichte von CD3+-Zellen am Invasionsrand (IM) und der Dichte von CD8+-Zellen am Invasionsrand (IM);
- b) Vergleichen von jedem in Schritt a) erhaltenen Wert mit einer Verteilung von Werten, die für jede der Dichten von einer Referenzgruppe von Patienten, die am Krebs leiden, erhalten wurden;
- c) Bestimmen für jeden in Schritt a) erhaltenen Wert für die Dichten des Perzentils der Verteilung, entsprechend den in Schritt a) erhaltenen Werten;
- d) Berechnen des arithmetischen Mittelwerts oder des Medianwerts des Perzentils
- e) Vergleichen des in Schritt d) erhaltenen arithmetischen Mittelwerts oder des Medianwerts des Perzentils mit einem vorbestimmten arithmetischen Referenzmittelwert oder einem vorbestimmten Medianwert des Perzentils, und
- f) Zuweisen einer "niedrigen", "mittleren" oder "hohen" Punktzahl, je nachdem, ob der arithmetische Mittelwert:
- niedriger (oder gleich) als der niedrigste vorbestimmte Referenzwert des arithmetischen Mittelwerts des Perzentils ist ("niedrig")
- zwischen den 2 vorbestimmten arithmetischen Referenzmittelwerten des Perzentils liegt ("mittel")
- höher als der höchste vorbestimmte arithmetische Referenzmittelwert des Perzentils ist ("hoch")
- g) mit der Schlussfolgerung, dass:
- wenn der Patient als "niedrig" eingestuft wird, das Risiko von Lymphknotenmetastasien und/oder Rezidiv hoch ist
- wenn der Patient als "mittel" eingestuft wird, das Risiko von Lymphknotenmetastasen und/oder Rezidiv mittel hoch ist
- wenn der Patient als "hoch" eingestuft wird, das Risiko von Lymphknotenmetastasen und/oder Rezidiv niedrig ist.

2. Verfahren nach Anspruch 1, wobei das Verfahren zum Vorhersagen von Zeit bis Rezidiv geeignet ist.

3. Verfahren nach Anspruch 1, wobei das Verfahren zum Vorhersagen der krankheitsfreien Überlebenszeit geeignet ist.

## Revendications

1. Procédé de prédiction du risque de métastase ganglionnaire et/ou de récidive chez un patient atteint d'un cancer colorectal T1 traité par résection endoscopique et présentant au moins un critère histologique défavorable choisi dans le groupe constitué d'une marge verticale positive, d'une faible différenciation, d'emboles tumoraux vasculaires ou lymphatiques, d'un bourgeonnement tumoral et d'une profondeur d'invasion sous-muqueuse > 1000 µm, comprenant :
- a) la quantification, dans un échantillon de tissu tumoral obtenu du patient, de la densité de cellules CD3+ au centre de la tumeur (CT), la densité de cellules CD8+ au centre de la tumeur (CT), la densité de cellules CD3+ dans la marge d'invasion (IM) et la densité de cellules CD8+ dans la marge d'invasion (IM) ;
- b) la comparaison de chaque valeur obtenue à l'étape a) à une répartition de valeurs obtenues pour chacune desdites densités à partir d'un groupe de référence de patients atteints dudit cancer ;
- c) la détermination, pour chaque valeur obtenue à l'étape a) pour lesdites densités, du percentile de la répartition auquel correspondent les valeurs obtenues à l'étape a) ;
- d) le calcul de la valeur moyenne arithmétique ou de la valeur médiane du percentile ;
- e) la comparaison de la valeur moyenne arithmétique ou de la valeur médiane du percentile obtenue à l'étape d) à une valeur moyenne arithmétique de référence prédéterminée ou à une valeur médiane prédéterminée du percentile, et
- f) l'attribution d'une note « faible », « intermédiaire » ou « élevée » selon que la valeur moyenne arithmétique :
- est inférieure (ou égale) à la valeur moyenne arithmétique de référence prédéterminée la plus basse du percentile (« faible »)
- est comprise entre les 2 valeurs moyennes arithmétiques de référence prédéterminées du percentile (« intermédiaire »)
- est supérieure à la valeur moyenne arithmétique de référence prédéterminée la plus élevée du percentile (« élevée »),
- g) la conclusion que :
- lorsque le patient est classé comme présentant un risque « faible », le risque de métastase ganglionnaire et/ou de récidive est élevé ;
- lorsque le patient est classé comme présentant un risque « intermédiaire », le risque de métastase ganglionnaire et/ou de récidive est intermédiaire ;
- lorsque le patient est classé comme présentant un risque « élevé », le risque de métastase ganglionnaire et/ou de récidive est faible.

2. Procédé selon la revendication 1, dans lequel le procédé est adapté à la prédiction du temps de récidive.

3. Procédé selon la revendication 1, dans lequel le procédé est adapté à la prédiction de la survie sans maladie.
